# EUROPÄISCHE PATENTSCHRIFT

(11) **EP 0 400 196 B1**
(45) Veröffentlichungstag und Bekanntmachung des Hinweises auf die Patenterteilung: **02.11.1994**
(21) Anmeldenummer: 89110039.8
(22) Anmeldetag: 02.06.1989
(51) Int. Cl.: A61B 17/22

(54) **Stosswellenkopf für die Zertrümmerung von Konkrementen**
Shock wave transducer for the destruction of concretions
Transducteur d'ondes de choc pour destruction de concrétions

(43) Veröffentlichungstag der Anmeldung: 05.12.1990
(73) Patentinhaber: SIEMENS AKTIENGESELLSCHAFT, 80333 München (DE)
(72) Erfinder: Iffländer, Helmut, Dipl.-Ing. (FH), D-8521 Spardorf (DE); Matura, Eike, Dipl.-Ing. (FH), D-8520 Erlangen (DE); Polster, Walter, Ing. grad., D-8520 Erlangen (DE); Rattner, Manfred, Ing., D-8521 Grossenseebach (DE)

(56) Entgegenhaltungen:
- EP-A- 0 316 863
- DE-A- 3 727 692

## Beschreibung

Die Erfindung betrifft einen Stoßwellenkopf gemäß dem ersten Teil des Patentanspruches 1.

Voraussetzung für die gezielte Zertrümmerung von Konkrementen mit einem Stoßwellenkopf, der einen Fokusbereich aufweist, auf den die Stoßwellen fokussiert sind, ist eine Ortung der Konkremente. Hierzu kann ein Röntgensystem mit Röntgenstrahler und Strahlenempfänger, aber auch ein Ultraschall-Ortungssystem vorgesehen sein, das sich insbesondere zur Ortung von Gallensteinen eignet. Das Ultraschall-Ortungssystem kann dabei zentrisch im Stoßwellenkopf angeordnet werden, so daß sein Zentralstrahl und der Zentralstrahl der vom Stoßwellenkopf ausgesandten Stoßwellen zusammenfallen. Ist zusätzlich zum Ultraschall-Ortungssystem noch ein Röntgensystem für die Ortung vorhanden, so ist es zweckmäßig, das Ultraschall-Ortungssystem entfernbar im Stoßwellenkopf anzuordnen, so daß der zentrische Bereich des Stoßwellenkopfes für die Ortung mit Röntgenstrahlen von diesen ungehindert durchdrungen werden kann.

Durch die EP-A-0 316 863 ist ein Stoßwellenkopf gemäß dem ersten Teil des Patentanspruches 1 bekannt, bei dem der hohlzylindrische Raum im Zentrum des Stoßwellenkopfes beidseitig offen ist, so daß das Ultraschall-Ortungssystem unmittelbar am Patienten anliegt.

Der Erfindung liegt die Aufgabe zugrunde, einen Stoßwellenkopf gemäß dem ersten Teil des Patentanspruches 1 so auszubilden, daß eine einheitliche Anlagefläche auf der Applikationsseite geschaffen wird.

Diese Aufgabe ist erfindungsgemäß gelöst durch die Merkmale des zweiten Teiles des Patentanspruches 1.

Die Erfindung ist nachfolgend anhand eines in der Zeichnung dargestellten Ausführungsbeispiels näher erläutert. Es zeigen:
- Fig. 1: einen Lithotripter zur Erläuterung des Erfindungsgedankens, und
- Fig. 2: den Stoßwellenkopf des Lithotripters gemäß Fig. 1.

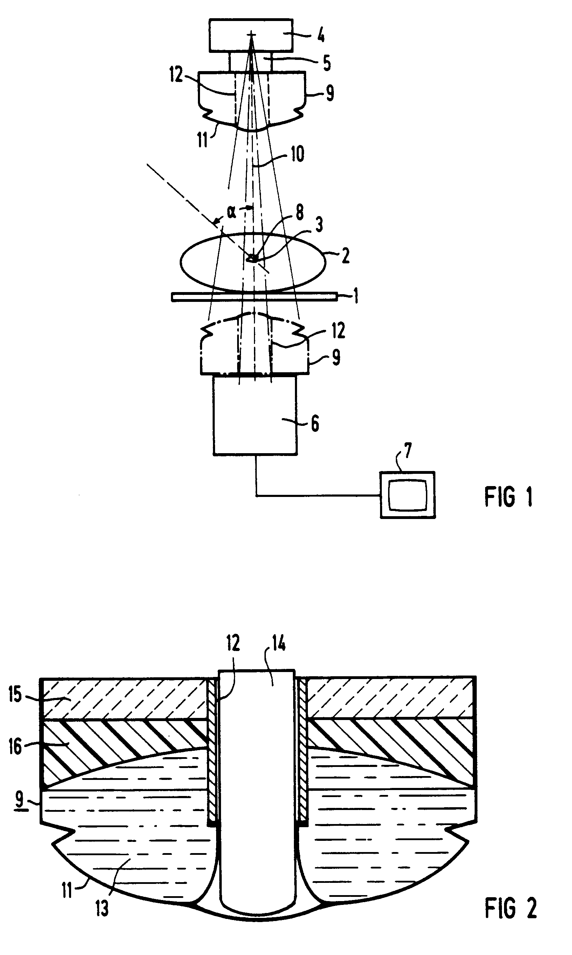

In der Fig. 1 ist eine Liege 1 dargestellt, auf der ein Patient 2 liegt, der ein Konkrement 3 enthält. Das Konkrement 3 soll durch Stoßwellen zertrümmert werden. Zur Ortung des Konkrements 3 ist ein Röntgensystem mit einem Röntgenstrahler 4 mit Primärstrahlenblende 5 und einem Röntgenbildverstärker 6 mit nachgeschalteter Fernsehkette 7 vorgesehen. Damit eine räumliche Information über die Lage des Konkrements 3 erhalten wird, kann das Röntgensystem 4, 5, 6 um eine Achse 8 um einen Winkel alpha geschwenkt werden, so daß eine Durchstrahlung des Patienten 2 unter verschiedenen Richtungen möglich ist. Die Achse 8 soll dabei mit dem Konkrement 3 zusammenfallen. Hierzu ist die Liege 1 entsprechend verstellbar.

Nach der Ortung und Einstellung des Konkrements 3 in der geschilderten Weise erfolgt die Beschallung des Konkrements 3 mit Stoßwellen. Hierzu ist an der Primärstrahlenblende 5 ein Stoßwellenkopf 9 angebracht, der auf einen Fokusbereich fokussierte Stoßwellen aussendet und so eingestellt wird, daß der Fokusbereich ebenfalls mit dem Konkrement 3 zusammenfällt. Hierzu kann der Stoßwellenkopf 9 in Richtung des Zentralstrahls 10 des Röntgensystems 4, 5, 6 verstellbar gelagert sein. Die Verstellung kann dabei individuell, aber auch zusammen mit dem Röntgenstrahler 4 erfolgen. Der Stoßwellenkopf 9 ist an seiner Applikationsseite von einer flexiblen Folie 11 abgedeckt, die zur akustischen Kopplung an der Oberfläche des Patienten 2 angelegt wird.

Wesentlich ist, daß der Stoßwellenkopf 9 in Bezug auf das Röntgensystem 4, 5, 6 derart angeordnet ist, daß der Zentralstrahl 10 des Röntgensystems 4, 5, 6 etwa zentrisch durch den Stoßwellenkopf 9 verläuft. Hierzu besitzt der Stoßwellenkopf 9 einen von der Röntgenstrahlung durchsetzten, zentrischen, hohlzylindrischen Raum 12 ohne Strahlenschwächung. Der Raum 12 ist luftgefüllt. Seine hohlzylindrische Wandung begrenzt den mit Koppelflüssigkeit gefüllten Raum des Stoßwellenkopfes 9. Dies ist nachfolgend anhand der Fig. 2 noch näher erläutert.

Bei dem in Fig. 1 dargestellten Lithotripter fällt der Zentralstrahl 10 des Röntgensystems 4, 5, 6 mit dem Zentralstrahl der von dem Stoßwellenkopf 9 ausgesandten Schallkeule zusammen. Die Ortung erfolgt durch Verschwenken des Röntgensystems 4, 5, 6. Es ist auch möglich, ein zweites Röntgensystem vorzusehen, das unter dem Winkel alpha gegenüber dem Zentralstrahl 10 einstrahlt, und das Röntgensystem 4, 5, 6 ortsfest anzuordnen. Ferner ist es möglich, den Röntgenstrahler 4 zur Anfertigung von Röntgen-Stereo-Bildern auszubilden, indem zwei wechselweise strahlende Foken vorgesehen werden, so daß die beiden Zentralstrahlen, die von diesen beiden Foken ausgehen, einen kleinen Winkel miteinander bilden. In diesem Fall muß der Raum 12 in seinen Abmessungen so ausgebildet werden, daß die von beiden Foken ausgesandten Röntgenstrahlenbündel voll den Patienten 2 durchsetzen.

Die Fig. 1 zeigt strichpunktiert eine zweite Möglichkeit der Anordnung des Stoßwellenkopfes 9 vor dem Röntgenbildverstärker 6. In diesem Fall ist das zur Bilderzeugung nutzbare Röntgenstrahlenbündel ebenfalls durch die Abmessungen des Raumes 12 festgelegt.

Die Fig. 2 zeigt, daß der Stoßwellenkopf 9 einen geschlossenen, von einer Koppelflüssigkeit, z.B. Wasser, ausgefüllten Raum 13 aufweist. Die Applikationsseite des Stoßwellenkopfes 9 ist von der flexiblen Folie 11 abgedeckt, die über den Raum 12 gezogen ist. Die hohlzylindrische Wandung des Raums 12 begrenzt den mit Koppelflüssigkeit gefüllten Raum. An der Rückseite des Raumes 13 ist eine Stoßwellenquelle 15 angeordnet.

Im Raum 12 ist ein Ultraschall-Ortungssystem 14 angeordnet, das in axialer Richtung aus dem Raum 12 herausgezogen werden kann. Der Raum 12 ist - wie oben ausgeführt - luftgefüllt und seine hohlzylindrische Wandung begrenzt den mit Koppelflüssigkeit gefüllten Raum 13, so daß beim Herausziehen des Ultraschall-Ortungssystems 14 keinerlei Maßnahmen gegen das Herauslaufen von Koppelflüssigkeit getroffen werden müssen.

Im Stoßwellenkopf 9 ist die Stoßwellenquelle 15 vorgesehen, die z.B. piezokeramischer oder elektrodynamischer Art sein kann. Sie sendet fokussierte Stoßwellen aus, die durch ein akustisches Linsensystem 16, das zur Veränderung der Lage des Fokusbereichs einstellbar sein kann, fokussiert wird.

## Patentansprüche

1. Stoßwellenkopf (9) für die Zertrümmerung von Konkrementen (3) im menschlichen Körper (2) mit einer Stoßwellenquelle (15) und einem geschlossenen, von einer Koppelflüssigkeit ausgefüllten Raum (13), wobei die Applikationsseite dieses Raumes (13) von einer flexiblen Folie (11) für die akustische Ankopplung am Behandlungsobjekt (2) abgeschlossen und zentrisch im Stoßwellenkopf (9) ein Ultraschall-Ortungssystem (14) angeordnet werden kann, und wobei für die Aufnahme des Ultraschall-Ortungssystems (14) ein hohlzylindrischer, luftgefüllter und somit nicht röntgenstrahlenschwächender Raum (12) vorgesehen ist, dessen Wandung den mit Koppelflüssigkeit gefüllten Raum (13) begrenzt, **dadurch gegekennzeichnet,** daß der hohlzylindrische Raum (12) auf der Applikationsseite von einer flexiblen Folie (11) abgeschlossen ist.

## Claims

1. Shock wave head (9) for the disintegration of concretions (3) in the human body (2), the head having a shock wave source (15) and a closed space (13) which is filled by a coupling fluid, wherein the application side of this space (13) can be closed by a flexible foil (11) for the acoustic coupling to the object (2) of treatment, and an ultrasound-location system (14) can be arranged centrally in the shock wave head (9), and wherein provided for the accommodation of the ultrasound-location system (14) there is a hollowly cylindrical space (12) which is air-filled and thus does not attenuate X-rays and the wall of which delimits the space (13) filled with coupling fluid, characterised in that the hollowly cylindrical space (12) is closed on the application side by a flexible foil (11).

## Revendications

1. Tête (9) de production d'ondes de choc pour la désagrégation de concrétions (3) dans le corps humain (2), comportant une source d'ondes de choc (15) et une chambre fermée (13) remplie par un liquide de couplage, et dans laquelle le côté d'application de cette chambre (13) est fermée par une feuille flexible (11) pour réaliser le couplage acoustique avec l'objet de traitement (2) et un système de localisation à ultrasons (14) peut être disposé en position centrée dans la tête (19) de production d'ondes de choc, et dans laquelle, pour loger le système (14) de localisation à ultrasons, il est prévu une chambre en forme de cylindre creux (12), qui est remplie d'air et n'affaiblit par conséquent pas le rayonnement X et dont la paroi limite la chambre (13) remplie par le liquide de couplage, caractérisée par le fait que la chambre en forme de cylindre creux (12) est fermée, sur le côté d'application, par une feuille flexible (11).
